(19) Europäisches Patentamt
European Patent Office
Office européen des brevets

(11) Veröffentlichungsnummer: **0 124 774**
**A1**

(12) **EUROPÄISCHE PATENTANMELDUNG**

(21) Anmeldenummer: **84103743.5**

(22) Anmeldetag: **05.04.84**

(51) Int. Cl.³: **A 61 K 33/18**
A 61 K 31/79
//(A61K33/18, 31/79, 31/765)

(30) Priorität: **07.04.83 DE 3312431**

(43) Veröffentlichungstag der Anmeldung:
**14.11.84 Patentblatt 84/46**

(84) Benannte Vertragsstaaten:
**AT BE CH DE FR GB IT LI LU NL SE**

(71) Anmelder: **Intermedicat GmbH**
**Gerliswilstrasse 45**
**CH-6020 Emmenbrücke(CH)**

(72) Erfinder: **Schröder, Jürgen, Dr.**
**Auf dem Rottheil 7**
**D-3509 Spangenberg(DE)**

(72) Erfinder: **Werner, Heinz Helmut, Dr.**
**Am Steig 2**
**D-3508 Melsungen(DE)**

(74) Vertreter: **von Kreisler, Alek, Dipl.-Chem. et al,**
**Deichmannhaus am Hauptbahnhof**
**D-5000 Köln 1(DE)**

(54) **Polyvinylpyrrolydon-Jod-Wundpulver mit synergistisch wirkender Pudergrundlage.**

(57) Die Erfindung betrifft antimikrobiell wirksame Wundpuder mit Polyvinylpyrrolidon-Iod als Wirkstoff, die dadurch gekennzeichnet sind, daß sie als Pudergrunglage feste Verbindungen, die bei der Polymerisation von Ethylenglykol bzw. Ethylenoxid und/oder Propylenoxid entstehen, zusammen mit für Puder üblichen weiteren Formulierungsstoffen enthalten.

EP 0 124 774 A1

VON KREISLER SCHÖNWALD EISHOLD FUES
VON KREISLER KELLER SELTING WERNER

0124774

PATENTANWÄLTE
Dr.-Ing. von Kreisler † 1973
Dr.-Ing. K. Schönwald, Köln
Dr.-Ing. K. W. Eishold, Bad Soden
Dr. J. F. Fues, Köln
Dipl.-Chem. Alek von Kreisler, Köln
Dipl.-Chem. Carola Keller, Köln
Dipl.-Ing. G. Selting, Köln
Dr. H.-K. Werner, Köln

B.Braun Melsungen AG
Melsungen

BEZEICHNUNG GEÄNDERT
siehe Titelseite

DEICHMANNHAUS AM HAUPTBAHNHOF
D-5000 KÖLN 1

AvK/IM

PVP-Iod-Wundpuder mit synergistisch wirkender Pudergrundlage

---

Die Erfindung betrifft einen antimikrobiell wirksamen Wundpuder mit PVP-Iod als Wirkstoff, der dadurch gekennzeichnet ist, daß er als Pudergrundlage feste Verbindungen enthält, die bei der Polymerisation von Ethylenglykol bzw. Ethylenoxid und/oder Propylenoxid entstehen,zusammen mit für Puder üblichen weiteren Formulierungsstoffen.

Die Behandlung großflächiger, infizierter, nässender und offener Wunden ist ein auch heute noch nicht völlig befriedigend gelöstes Problem. Generell werden für die Behandlung derartiger Wunden Salben oder Puder eingesetzt.

Antimikrobielle Salben zur Wundbehandlung nach dem Stand der Technik enthalten z.B. als antimikrobielle Wirkstoffe Antibiotika, Sulfonamide und Antiseptika. Stellvertretend für diese Stoffgruppen seien genannt: Neomycin, Silbersulfadiacin und PVP-Iod. Diese Wirkstoffe werden mit den verschiedenen handelsüblichen Salbengrundlagen zu entsprechenden Fertigprodukten verarbeitet, wobei als Salbengrundlagen sowohl verschiedene vollsynthetische, fettfreie Salbengrundlagen des Marktes dienen können, als auch

Telefon: (0221) 131041 · Telex: 888 2307 dopa d · Telegramm: Dompatent Köln

seit langem bekannte fetthaltige Salbengrundlagen. Auch für den antiseptischen Wirkstoff PVP-Iod sind sowohl Salbenzubereitungen in fettfreier als auch fetthaltiger Salbengrundlage auf dem Markt zu finden.

Die Wundbehandlung mit Pudern hat gegenüber einer Behandlung mit Salben den gewichtigen Vorteil, daß keine mechanische Manipulation auf der Wunde erforderlich ist.

Als Pudergrundlagen, welche in der Wundbehandlung eingesetzt werden können, werden in der Literatur resorbierbare Stärke und Milchzucker genannt. Außerdem ist als Mittel zur Wundbehandlung der Einsatz von hochmolekularem Dextran, bekannt z.B. unter dem Markennamen DEBRISORB$^R$ üblich. Weiterhin wird in der Literatur (GB-PS 2 084 464) der Einsatz eines Puders, bestehend aus Milchzucker und dem Antiseptikum PVP-Iod, beschrieben. Ein besonderer Effekt durch die Kombination des an sich bekannten Milchzuckers mit dem ebenfalls bekannten Antiseptikum PVP-Iod wird jedoch nicht genannt.

Polyvinylpyrrolidon (PVP) ist ein Polymerisat der allgemeinen Formel (I),

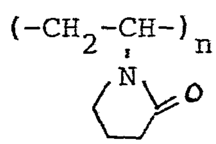

(I)

das in wässrigen Lösungen proteinähnlichen Charakter hat und Komplexe mit verschiedenen organischen und anorganischen Verbindungen bildet. Sein Komplex mit Iod dient als Desinfizienz für innere und äußere Anwendung.

PVP-Iod gehört zu den sog. Iodophoren. Dies sind Substanzen, welche eine größere Menge Iod als Depotwirkstoff komplex gebunden enthalten. und im Gleichgewicht jeweils nur eine sehr geringe Menge Iod abgeben. Dadurch ist einerseits die seit langem bekannte umfassende antimikrobielle Wirkung des Iod gegeben, während andererseits die extrem starken Nebenwirkungen früherer iodhaltiger Zubereitungen, wie Lugol'sche Lösung oder Iodine Solution USP, vermieden werden. Diese Nebenwirkungen äußern sich in extremen Schmerzreaktionen bei der Anwendung und in starken Gewebereizungen.

In Abwandlung einer in der Literatur beschriebenen Testmethode (M.Loran u. K.Kleinmann, Am.J.Hosp.Pharm. 32, 431 (1975))lässt sich der Unterschied in der Komplexbindung des Iod zwischen einer Iodine Solution USP und einer PVP Iod-Lösung mit folgender Versuchsanordnung demonstrieren:

Die zu prüfende Lösung wird in ein Zentrifugenglas gegeben, darüber wird ein KI-Stärke-Papier befestigt. In Abhängigkeit vom Iod-Dampfdruck der zu untersuchenden Lösung verfärbt sich das KI-Stärke-Papier mit unterschiedlicher Geschwindigkeit und Intensität. Der Iod-Dampfdruck über der zu prüfenden Lösung ist ein gutes Maß für den Umfang der Komplexierung des Iod. Prüft man mit dieser Versuchanordnung eine Iodine Solution USP, so ist eine Blaufärbung des Papiers bereits nach 90 Sekunden deutlich sichtbar, während eine PVP-Iod-Lösung mit gleicher Konzentration an verfügbarem Iod erst nach 170 Minuten eine Verfärbung zeigt.

Die vorliegende Erfindung geht nun davon aus, daß sich die Nebenwirkungen des PVP-Iod weiter verringern lassen, wenn man eine speziell dem Wirkstoff PVP-Iod angepasste Pudergrundlage findet, welche die Bindung des Iod im Polyvinylpyrrolidon-Komplex weiter verstärkt. Für die Wundbehandlung sollte die Pudergrundlage weiterhin vollständig löslich in Wasser sein, damit sich der Wundpuder im Wundsekret auflöst und keine Manipulation an der Wunde zur Entfernung des verbrauchten Puders, wie bei der Anwendung von Dextran oder resorbierbarer Stärke, erforderlich ist.

Aufgabe der vorliegenden Erfindung ist es, eine diesen Anforderungen genügende Pudergrundlage für das gut wirksame Antiseptikum PVP-Iod zu entwickeln.

Überraschenderweise wurde nun gefunden, daß feste Polyethylenglykole bzw. Ethylenoxid-Propylenoxid-Blockpolymerisate eine ideale Pudergrundlage für den antimikrobiellen Wirkstoff PVP-Iod darstellen. Diese Verbindungen sind vollständig wasserlöslich und erfüllen daher die Forderung nach rückstandsfreier Auflösung im Wundsekret.

Unter festen Polyethylenglykolen bzw. EO/PO-Blockpolymerisaten sind zunächst bei Zimmertemperatur feste Polymerisate im Molekulargewichtsbereich = 1500 und = 5000 EO/PO-Blockpolymerisate zu verstehen, die für die Herstellung der Wundpuder Verwendung finden.

Es ist weiterhin möglich, in diese festen, in Pulverform vorliegenden Polymere kleine Mengen gleichartiger Polymerer, welche bei Zimmertemperatur in flüssiger Form vorliegen, einzuarbeiten, ohne daß die Pulvereigenschaft des Gemisches durch Pastenbildung verloren geht. So kann man z.B. eine Schmelze aus 8 Teilen Polyethylenglykol 8000 und 1 Teil Polyethylenglykol 400 nach dem Erkalten mahlen, wobei ein streufähiges Pulver entsteht. In die Schmelze aus Polyethylenglykolen und/oder EO/PO-Blockpolymerisaten können auch andere wasserlösliche Verbindungen wie z.B. PVP oder Sorbit, eingearbeitet werden.

Überraschenderweise wird jedoch außerdem die Komplexbindung des Iod im Polyvinylpyrrolidon-Komplex durch den Zusatz von Polyethylenglykol bzw. Ethylenoxid-Propylenoxid-Blockpolymerisaten deutlich verbessert.

Dieses Ergebnis ist insofern besonders unerwartet und überraschend, als zwar in der Literatur (R. Springer, R. Lang, Apotheker-Zeitung, 108.Jahr., Nr. 42, 1605 (1968)) Komplexe zwischen vorzugsweise flüssigem Polyethylenglykol und Iod beschrieben sind. Unter Verwendung der oben beschriebenen Versuchsanordnung läßt sich jedoch zeigen, daß der Iod-Dampfdruck über einen 10 %igen PVP-Iod-Lösung (enthaltend 8,5 % PVP, 1 % $I_2$ und 0,5 % $I^-$) praktisch genauso groß ist wie der Iod-Dampfdruck einer Lösung, die 15 % Polyethylenglykol, 1 % $I_2$ und 0,5 % $I^-$ enthält. Dies bedeutet, daß die Einbindung des Iod in einen Polyvinylpyrrolidon-Komplex in einer 8,5 %igen PVP-Lösung und die Einbindung des Iod in einen Polyethylenglykol-Komplex in einer 15 %igen Polyethylenglykol-Lösung in etwa gleich gut sind. Nach den bekannten Grundsätzen der Komplexbildung ist es nun nicht zu erwarten, daß die Anwesenheit von zwei annähernd gleich starken Komplexbildnern zu einer deutlichen Verbesserung der Einbindung in einen Komplex führt.

Werden wässrige Lösungen, in denen $I_3^-$ bzw. $I_2$ und $I^-$ durch Komplexierung mit Polyethylenglykol gelöst ist, stark verdünnt, so wird wieder elementares Iod frei. Derartige wässrige Iod-Lösungen werden daher in der Praxis nicht eingesetzt.

Im Gegensatz dazu fällt in PVP-Iod-Lösungen mit zunehmender Verdünnung kein Iod aus. Diese Tatsache läßt eine Verstärkung der komplexen Bindung des Iod im Polyvinylpyrrolidon-Komplex bei Zusatz von Polyethylenglykol ebenfalls überraschend erscheinen.

Prüft man mit der beschriebenen Versuchsanordnung beispielsweise den Dampfdruck einer 10 %igen PVP-Iod-Lösung (Lösungsmittel Ethanol/$H_2O$ im Verhältnis 1 : 1), so verfärbt sich das KI-Stärke-Papier aufgrund der unter diesen Bedingungen sukzessive frei werdenden Ioddämpfe. Der Zusatz von steigenden Mengen Polyethylenglykol (z.B. 15 oder 30 %) mit einem mittleren Molekulargewicht von 4000 führt unter gleichen Prüfbedingungen zu einer deutlich geringeren Verfärbung aufgrund eines niedrigeren Iod-Dampfdruckes über den polyethylenglykolhaltigen Lösungen. Dies ist mit einer verstärkten Bindung des Iods im Polyvinylpyrrolidon-Komplex durch den Zusatz von Polyethylenglykol zu erklären. Der Gesamtgehalt an elementarem Iod in beiden Lösungen ist gleich, wie durch Titration mit Natriumthiosulfat nachgewiesen werden kann.

Die Erfindung wird durch die nachfolgenden Beispiele näher erläutert.

Die Vorteile der erfindungsgemäßen Produkte ließen sich mit der oben beschriebenen Versuchsanordnung demonstrieren:

Die erfindungsgemäßen Wundpuder aus den nachfolgenden Beispielen 1 und 4 wurden in Wasser gelöst, so daß eine 50 %ige Lösung entstand. Prüfte man diese Lösungen bezüglich ihres Iod-Dampfdruckes nach der bereits beschriebenen Versuchsanordnung, so zeigte sich nur eine geringe Verfärbung des KJ-Stärkepapiers. Bei der analogen Prüfung eines bereits bekannten Puders aus 10 % PVP-Iod und 90 % Lactose nach der GB-PS 2 084 464 zeigten sich folgende Nachteile:
Bei dem Versuch, eine 50 %ige wässrige Lösung herzustellen, ging das gesamte PVP-Iod schnell in Lösung, während ein Teil der Lactose aufgrund der geringeren Löslichkeit als weißer Bodenkörper zurückblieb.

Dieses Verhalten der bekannten Puderzubreitung war nicht überraschend, da diese nur durch mechanisches Mischen der trockenen pulverförmigen Komponenten hergestellt wurde und nicht im Hinblick auf optimales Löslichkeitsverhalten auf der nässenden, sekretierenden Wunde konzipiert wurde.

Auf die offene Wunde übertragen bedeutet dies, daß der PVP-Iod-Anteil des Puders schnell in Lösung geht und mit dem Wundsekret zu antimikrobiell unwirksamem Iodid reagiert, während noch ungelöste Lactose auf der Wunde vorhanden ist.

Die erfindungsgemäßen Puder zeigten aufgrund der gleichzeitigen homogenen Verflüssigung demgegenüber keine unterschiedlichen Lösegeschwindigkeiten der einzelnen Inhaltsstoffe, wodurch das antimikrobiell wirksame Iod über einen längeren Zeitraum abgegeben wurde und damit länger verfügbar war.

Prüfte man den Iod-Dampfdruck der 50 %igen wässrigen Lösung mit Lactose-Bodenkörper des bekannten Puders nach der bereits bekannten Versuchsanordnung, so zeigte sich eine erheblich stärkere Verfärbung des KJ-Stärkepapiers als bei der Prüfung der erfindungsgemäßen Puder.

Beispiel 1

Ein erfindungsgemäßer Wundpuder wird wie folgt hergestellt:
90 kg Polyethylenglykol (PEG) mit einem mittleren Molekulargewicht von 4000 werden geschmolzen. In diese Schmelze werden 10 kg PVP-Iod mit einer maximalen Korngröße von 10 µ eingerührt und dispergiert. Nach dem Erkalten wird die erstarrte Masse gemahlen, wobei die

Mahlfeinheit mit bekannter Technik so gesteuert wird, daß die minimale Korngröße über der maximalen Korngröße der PVP-Iod-Partikel liegt. Dadurch wird erreicht, daß die PVP-Iod-Partikel mit PEG umhüllt sind.

Beispiel 2

Ein Gemisch aus 32 kg PEG 1500, 32 kg PEG 8000 und 32 kg PEG 20 000 wird geschmolzen. In diese Schmelze werden 4 kg PVP-Iod eingearbeitet. Die weitere Verarbeitung erfolgt wie in Beispiel 1.

Beispiel 3

2 kg PVP-Iod werden in 10 kg PEG 400 gelöst. Diese Lösung wird in eine Schmelze von 88 kg PEG 8000 eingerührt. Nach dem Erkalten wird die erstarrte Schmelze gemahlen.

Beispiel 4

70 kg PEG 4000 werden geschmolzen. In diese Schmelze werden 20 kg PVP sowie 10 kg PVP-Iod mit einer maximalen Korngröße von 10 µ eingerührt und dispergiert. Die weitere Verarbeitung erfolgt wie in Beispiel 1.

Beispiel 5

70 kg eines EO-PO-Blockpolymerisates mit einem mittleren Molekulargewicht von 12 500 bei einem Molekulargewichtsanteil des Polypropylenglykol von 4000 werden geschmolzen. In die Schmelze werden 30 kg PVP-Iod mit einer maximalen Korngröße von 10 µ eingerührt und dispergiert. Die weitere Verarbeitung erfolgt wie in Beispiel 1.

Patentansprüche

1. Antimikrobiell wirksamer Wundpuder mit Polyvinylpyrrolidon-Iod (PVP-Iod) als Wirkstoff, dadurch gekennzeichnet, daß er als Pudergrundlage feste Verbindungen, die bei der Polymerisation von Ethylenglykol bzw. Ethylenoxid und/oder Propylenoxid entstehen, zusammen mit für Puder üblichen weiteren Formulierungsstoffen enthält.

2. Antimikrobiell wirksamer Wundpuder nach Anspruch 1, dadurch gekennzeichnet, daß das Polyethylenglykol ein mittleres Molekulargewicht von 4000 aufweist.

3. Antimikrobiell wirksamer Wundpuder nach Anspruch 1, dadurch gekennzeichnet, daß das feste Polyethylenglykol oder EO/PO-Blockpolymerisat einen Anteil flüssiges Polyethylenglykol mit einem mittleren Molekulargewicht von 200 bis 1 000 enthält.

4. Antimikrobiell wirksamer Wundpuder nach Anspruch 1, dadurch gekennzeichnet, daß die Ethylenoxid-Propylenoxid-Blockpolymerisate ein mittleres Molekulargewicht zwischen 5 000 und 14 000 aufweisen.

5. Antimikrobiell wirksamer Wundpuder nach Anspruch 1, dadurch gekennzeichnet, daß das feste Polyethylenglykol oder EO/PO-Blockpolymerisat einen Anteil flüssiges oder pastöses EO/PO-Blockpolymerisat mit einem mittleren Molekulargewicht von 1000 bis 5000 enthält.

6. Verwendung von festen Polymerisaten von Ethylenglykol bzw. Ethylenoxid und/oder Propylenoxid als Pudergrundlage für antimikrobiell wirksame Wundpuder mit Polyvinylpyrrolidon-Iod (PVP-Iod) als Wirkstoff.

Europäisches Patentamt

**0124774**

Nummer der Anmeldung

# EUROPÄISCHER RECHERCHENBERICHT

EP 84 10 3743

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | FR-A-2 267 112 (SUTURES, INC.) * Seite 16, Zeile 29 - Seite 17, Zeile 4; Ansprüche 7-11 * | 1-6 | A 61 K 33/18<br>A 61 K 31/79 //<br>(A 61 K 33/18<br>A 61 K 31/79<br>A 61 K 31/765) |
| X | FR-A-2 138 295 (MUNDIPHARMA AG) * Seite 30, Zeilen 1-25; Ansprüche 1,2; Seiten 28-29, Beispiel 14; Seite 5, Zeile 11 - Seite 6, Zeile 29; Seite 17, Zeilen 29-35 * | 1-6 | |
| A | CHEMICAL ABSTRACTS, Band 93, Nr. 20, 17. November 1980, Seite 361, Nr. 192048m, Columbus, Ohio, USA; & RO - A - 65 709 (INTREPRINDEREA DE ANTIBIOTICE) 15.12.1978 * Zusammenfassung * | 1-6 | |
| | | | RECHERCHIERTE SACHGEBIETE (Int. Cl. ³) |
| D,A | GB-A-2 084 464 (BETA MEDICAL PRODUCTS LTD.) | 1-6 | A 61 K |

Der vorliegende Recherchenbericht wurde für alle Patentansprüche erstellt.

| Recherchenort | Abschlußdatum der Recherche | Prüfer |
|---|---|---|
| DEN HAAG | 17-07-1984 | BRINKMANN C. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82